# EUROPEAN PATENT APPLICATION

(11) **EP 1 679 095 A1**
(43) Date of publication of application: **12.07.2006**
(21) Application number: 04792505.2
(22) Date of filing: 15.10.2004
(51) Int. Cl.: A61M 29/00

(54) **VESSEL STENT FEEDER**

(30) Priority: 15.10.2003 JP 2003355358
(71) Applicant: KABUSHIKIKAISHA IGAKI IRYO SEKKEI, Kyoto-shi, Kyoto 607-8035 (JP)
(72) Inventor: IGAKI, Keiji, Kabushikikaisha Igaki Iryo Sekkei, Yamashina-ku, Kyoto-shi, Kyoto 607-8035 (JP)
(74) Representative: Hoffmann, Jörg Peter
(86) International application number: PCT/JP2004/015288
(87) International publication number: WO 2005/037360

(57) **Abstract**

A device for delivery of a stent for a vessel, used for implanting a stent for the vessel 3 in the vessel, is disclosed. The device for delivery of a stent for the vessel includes a catheter (1) for insertion into the vessel of a living body, a balloon (2) mounted on an outer peripheral surface of the distal end side of the catheter and inflatable with a fluid supplied to the catheter, a stent for the vessel (3), formed of a biodegradable polymer to a tube form, mounted on the balloon in a diameter-contracted state and having self-expanding properties, and a stent holding member (21) formed of a polymer material to a tube form for covering at least a portion of the stent for the vessel from the catheter for holding the stent for the vessel on the balloon. The stent holding member has been drawn in the longitudinal direction. In the distal end of the stent holding member, located towards the distal end of the catheter, there is formed a tearing assisting portion (22). The stent holding member is torn along the tearing assisting portion, by expansion of the stent for the vessel attendant on inflation of the balloon, to release its holding of the stent for the vessel to enable expansion of the stent for the vessel (3).

## Description

### Technical Field

This invention relates to a device for delivery of a stent for a vessel, in which a stent for a vessel of a living body, such as blood vessel, trachea, bile duct or urethra, implanted in the vessel of the living body to support the inner lumen of the vessel from the inside, is held on a balloon provided to a catheter inserted into the vessel of the living body. More particularly, this invention relates to a device for delivery of a stent for the vessel, in which the stent for the vessel may be delivered to a targeted site of implantation in the vessel as the stent for the vessel is maintained mounted on the balloon provided to the catheter.

The present application claims priority based on the Japanese Patent Application 2003-355358 filed in Japan on October 15, 2003, the entire contents of which are incorporated herein by reference.

### Background Art

Where the state of stenosis has occurred in the vessel of a living body, such as a blood vessel of a living body, the technique of percutaneous transluminal angioplasty (PTA) is routinely applied. This is the procedure of introducing a balloon mounted to the vicinity of the distal end of a catheter to a site of stenosis, with the balloon then being expanded to hold open the site of stenosis to secure the blood flow.

However, it is known that, in a site where stenosis in the blood vessel has occurred, acute occlusion by the dissection of the intima, or re-stenosis, that is, re-narrowing of the once stenosis site of the blood vessel, tends to be produced at a high probability after PTA application.

For preventing such acute occlusion or re-stenosis, the technique of stent implantation is used. The technique consists in implanting a tubular stent in the site where PTA has been applied. The stent used is introduced into the blood vessel in a diameter-contracted state and subsequently expanded in diameter so as to be implanted in the blood vessel to support the wall of the blood vessel from the inside.

Up to now, a metal stent has been used as a stent implanted in the blood vessel. The metal stent may be classified into a balloon-expandable stent and a self-expandable stent.

The balloon-expandable stent is introduced to a targeted implant site in the blood vessel, in the diameter-contracted state, and subsequently expanded with inflation of the balloon. As the stents of this sort, there is a stent comprising a fine tube of stainless steel in which numerous slits are formed using e.g. a laser cutter to permit dilation of the stent, and a stent comprising metal filaments knitted to a tube form, as disclosed in the United States Patent 4,950,227.

The self-expandable stent is contracted in diameter under application of an external pressure and is introduced to the targeted implant site in the blood vessel in this diameter-contracted state. When the external pressure is subsequently removed, the self-expandable stent is spontaneously set to the expanded state to support the blood vessel from the inside. This sort of the self-expandable stent may be exemplified by a linear filament of metal, wound spirally and formed to a tube form, as disclosed in JP Laid-open Patent Publication Hei2-68052.

A device for delivery of a stent is used for implanting the above-described stent for the vessel in a targeted site in the blood vessel of a living body. The configuration of the device for delivery of a stent differs depending on the sort of the stent to be delivered, that is, depending on whether the stent is of the balloon-expandable type or the self-expandable type.

The stent delivery device for delivering the balloon-expandable stent in the blood vessel includes a catheter introduced into the blood vessel, and a balloon mounted in a diameter-contracted state to the distal end of the catheter. On the balloon is mounted a stent in the diameter-contracted state. The stent mounted on the balloon is pressed from the outer peripheral side and maintained so as not to be dropped out from the balloon. By progressively introducing the catheter into the blood vessel, the stent, mounted on the balloon, may be delivered as far as the targeted implant site in the blood vessel. The stent, delivered to the targeted implant site in the blood vessel, is expanded in diameter, by plastic deformation, with inflation of the balloon, such as to support the wall of the blood vessel from the inside.

It is basically sufficient only if the stent delivery device, used for implanting the balloon-expandable stent in the blood vessel, includes the configuration of mounting the stent in the diameter-contracted state on the balloon provided to the catheter.

As for the stent delivery device, used for delivering the balloon-expandable stent, there has also been made a proposal for providing a sheath which covers up the stent mounted to the balloon. The sheath used is provided for prohibiting the stent, mounted on the balloon, from being dropped out from the balloon.

On the other hand, the stent delivery device, used for delivering the self-expandable stent, within the blood vessel, includes a catheter, mounted with a stent thereon in the diameter-contracted state, and which is introduced into a protective sheath. The stent, mounted to the catheter in the diameter-contracted state, is covered up by the protective sheath so as to be thereby maintained in the diameter-contracted state. For implanting the stent in a targeted implant site in the blood vessel, using the above-described stent delivery device, a catheter, mounted with the stent thereon, is introduced as far as the targeted site of implantation in the blood vessel, along with the protective sheath. The catheter is then fixed, and only the protective sheath is pulled back within the blood vessel to release the stent mounted to the distal end of the catheter from the protective sheath. The stent, thus released from the protective sheath, is self-expanded by elasticity proper to the stent itself and expanded to a diameter capable of supporting the inner wall of the blood vessel.

The stent delivery device, used for implanting the self-expandable stent in the blood vessel, includes a catheter, mounted thereon with a stent in the diameter-contracted state, and a protective sheath for accommodating therein the catheter mounted with the stent, there being no necessity to provide a balloon for expanding the stent.

Meanwhile, there lacks up to now a therapeutic method for such a case in which re-stenosis has occurred in a site where angioplasty has once been applied and where there has been implanted a metal stent.

On the other hand, if metal, inherently a foreign substance to the living body, is left in the living body for an extended period of time, there is a risk that the blood vessel is affected by, for example, intimal hyperplasia produced in the site of implantation of the stent.

With a view to solving the problem inherent in the metal stent, so far used, the present Assignee has proposed a stent formed of a biodegradable polymer (see United States Patent 6,045,568, JP Patent 2842943, WO00/13737).

The stent, formed of the biodegradable polymer, may be absorbed into the tissue of the blood vessel after a preset time has elapsed as from the time of implantation in the blood vessel, such that the function thereof for supporting the blood vessel from an inner side is no longer needed, for example, after lapse of, for example, six to nine months. Since the stent of this sort may be caused to disappear in vivo, it is possible to suppress adverse effects due to the stent being foreign substance to the living body left for a prolonged time.

In particular, the present Assignee has proposed a stent for the vessel, prepared by knitting a yarn of a biodegradable polymer to a tube form (see United States Patent 6,045,568) and a stent for the vessel formed of a yarn of a biodegradable polymer which is arranged in a tube form in a non-woven non-knitted design (JP Patent 2842943). The present Assignee has also proposed a stent for the vessel in which the yarn formed of a biodegradable polymer is wound to produce a stent of a tube form, as the yarn is bent in a zigzag design, and in which the stent is expanded or contracted in diameter with the bends of the yarn as the displacing portions (WO00/13737), and has conducted an experiment of actually implanting the stent in the living body.

The stent, formed of the biodegradable polymer, is formed to a tube, and subsequently heat-set, by way of heat treatment, so as to be shape-memorized to a targeted outer diameter. The heat-setting is carried out at a temperature not lower than the glass transition temperature and not higher than the melting temperature of the biodegradable polymer which makes up the stent. The stent, shape-memorized to a target outer diameter, in readiness for implantation in the blood vessel, is contracted in diameter for insertion into the blood vessel. This contraction in diameter of the stent is done as an external pressure is applied to the stent, with or without heat setting. Here, the heat setting is carried out at a temperature lower than the temperature for heat setting carried out for maintaining the expanded configuration of the stent.

For expanding the stent, formed of a biodegradable polymer, a balloon expanding method, employing a balloon, is used. This method is used for quickly and reliably expanding the stent, inserted in the diameter-contracted state as far as the implant site in the blood vessel, to a stent size capable of supporting the inner wall of the blood vessel. Meanwhile, the stent, formed of the biodegradable polymer, may be afforded, on heating, with the properties of self-expansion, that is, the shape memorizing properties. The stent, formed of the biodegradable polymer, is self-expanded when it is mounted on a catheter and inserted in this state into the blood vessel of the living body so as to be heated by body temperature. Since the stent has the self-expanding properties, it is tightly contacted with the inner wall of the blood vessel to maintain the force of dilating the blood vessel from the inside for a preset time.

That is, the stent, formed of the biodegradable polymer, has the properties of self-expansion, despite the fact that it needs to be expanded with the aid of a balloon. For introducing and implanting this sort of the stent in the blood vessel, it is necessary to provide, along with a balloon for expanding the stent, an expansion prohibiting member for controlling the self-expansion of the stent caused by heating with body temperature when the stent is introduced into the blood vessel. That is, for preventing such accident in which the stent in the diameter-contracted state is inserted into the blood vessel and self-expanded, such that the stent is dropped out from the balloon, it is necessary to provide a protective sheath, restraining the self-expansion of the stent, mounted on the balloon.

There is also a probability that a stent, formed of a biodegradable polymer, and thus exhibiting the self-expanding properties, is gradually freed from the restraint by the protective sheath, after delivery to the targeted implant site in the blood vessel in the living body, such that, when a preset portion of the stent has been protruded from the protective sheath, the stent may jump up from inside the protective sheath, by its force of dilation, with the result that the stent is ultimately dropped out from the catheter. Hence, there is a risk that not only the stent cannot be correctly implanted at the targeted implant site in the blood vessel, but also the stent cannot be expanded by the balloon.

### Disclosure of the Invention

### Problems to be solved by the invention

It is an object of the present invention to provide a device for delivery of a stent for the vessel in which a stent for the vessel, which is formed of a biodegradable polymer and hence has the self-expanding properties, but still needs expansion by a balloon, may reliably be maintained in its position of placement on the balloon.

It is another object of the present invention to provide a device for delivery of a stent for the vessel in which a stent for the vessel, which is formed of a biodegradable polymer and hence has the self-expanding properties, but still needs expansion by a balloon, may correctly be implanted at a targeted implant site in the vessel.

It is a further object of the present invention to provide a device for delivery of a stent for the vessel in which a stent for the vessel, which is formed of a biodegradable polymer and hence has the self-expanding properties, but still needs expansion by a balloon, may reliably be retained on the balloon despite a simplified structure of the device.

For accomplishing the above objects, the present invention provides a device for delivery of a stent for a vessel having a catheter for insertion into the vessel of a living body, a balloon mounted on an outer peripheral surface of the distal end side of the catheter and inflatable with a fluid supplied to the catheter, a stent for a vessel mounted on the balloon in a diameter-contracted state, being formed of a biodegradable polymer to be a tube form and having self-expandable properties, and a stent holding member formed of a polymer material to a tube form for holding the stent for the vessel on the balloon, and configured for covering at least a portion of the stent for the vessel from the catheter. The stent holding member has been drawn in the longitudinal direction and is provided with a tearing assisting portion at a distal end thereof located towards the distal end of the catheter.

This tearing assisting portion is constituted by an incision provided to the distal end of the stent holding member. The incision is formed for extending along the drawing direction of the stent holding member.

Preferably, the stent holding member, employed in the present invention, is formed of PTFE (polytetrafluoroethylene), having highly lubricious properties, in view of ease in introducing the stent holding member into the vessel of the living body.

The stent holding member is carried against inadvertent movement relative to the catheter, even at the time of inflation of the balloon, by having its proximal side secured to the proximal side of the catheter. An air-vent through-hole is bored in the proximal side of the stent holding member secured to the catheter.

The stent holding member may cover up the stent for the vessel in its entirety. In this case, the distal end of the stent holding member, which covers up the stent for the vessel, is contracted in diameter to facilitate introduction thereof into the vessel of the living body.

The stent holding member may be connected to a yarn passed through the catheter and which is pulled out from a mid portion of the catheter. The stent holding member may then be released from the stent for the vessel by pulling the yarn outward from the catheter.

The stent for the vessel, retained by the holding member of the present invention, may be formed by, for example, a yarn of a biodegradable polymer, arranged in a tubular configuration.

With the device for delivery of a stent for the vessel, according to the present invention, in which the stent for the vessel, mounted on a balloon, provided to the catheter, is covered up by a stent holding member in the tube form, it is possible to prevent the stent for the vessel from being released from the balloon, while it is also possible to introduce and deliver of the stent for the vessel in the vessel of the living body as the mounted state of the stent for the vessel on the balloon is maintained.

When the force is applied to the stent holding member, covering up the stent for the vessel, in a direction of enlarging the diameter of the stent holding member as a result of inflation of the balloon, the stent holding member is torn, with the tearing assisting portion of the distal end thereof as a guide, thereby releasing the holding of the stent for the vessel. Hence, the stent for the vessel may reliably be expanded in diameter, in keeping with the inflation of the balloon, at the same time as positive retention of the stent for the vessel is achieved on the baloon.

In particular, in the device for delivery of a stent for the vessel, according to the present invention, the stent holding member has been drawn in the longitudinal direction, and hence may readily be torn, with inflation of the balloon, beginning from the tearing assisting portion, along the longitudinal direction, to permit the stent for the vessel to be expanded in diameter as the balloon is expanded.

Since the stent holding member has its proximal end secured to the catheter, it is not dropped out from the catheter, even after the tearing. In addition, since the stent holding member is formed of a highly lubricious material, it may be easily taken outward from within the vessel of the living body.

### Brief Description of the Drawings

Fig.1 is a perspective view showing an embodiment of a device for delivery of a stent for a vessel according to the present invention.
Fig.2 is a cross-sectional view, taken along line II-II of Fig.1, for illustrating a catheter used in the present invention.
Figs.3 is a cross-sectional side view for illustrating the state in which the stent for the vessel is mounted on a catheter and held by a stent holding member.
Fig.4 is a cross-sectional view, taken along line IV-IV of Fig.3, for illustrating the state in which the stent for the vessel is mounted on the catheter and held by the stent holding member.
Fig.5 is a perspective view showing an exemplary stent for the vessel used in the present invention.
Fig.6 is a perspective view showing an exemplary stent holding member.
Fig.7 is a partial side view showing another exemplary stent holding member.
Fig.8 is a partial perspective view showing a further exemplary stent holding member.
Fig.9 is a cross-sectional side view showing the state in which the stent holding member is secured to the catheter.
Fig.10 is a cross-sectional side view showing a stent holding member having an air vent through-hole bored therein.
Fig.11 is a cross-sectional side view showing the state in which air within the stent holding member is removed by way of evacuation.
Fig.12 is a side view showing the state in which the balloon is expanded to tear the stent holding member.
Fig.13 is a side view showing the state in which the balloon is inflated to expand the diameter of the stent for the vessel.
Fig.14 is a side view showing another embodiment of a device for delivery of a stent for the vessel according to the present invention, in which a maneuvering wire is provided for pulling out the stent holding member.
Fig.15 is a side view showing the state in which the stent for the vessel in the device for delivery of the stent for the vessel of Fig.14 has been expanded in diameter.
Fig.16 is a side view showing a further embodiment of the device for delivery of a stent for the vessel according to the present invention, in which the maneuvering wire provided for pulling out the stent holding member is passed through a protective sheath.

### Best Mode for Carrying out the Invention

In the following, a device for delivery of a stent for the vessel, according to the present invention, will be explained with reference to the drawings.

The device for delivery of a stent for the vessel, according to the present invention, is used for delivering the stent for the vessel, which is to be implanted in a vessel of a living body, such as blood vessel, trachea, bile duct or urethra, and which is used for supporting the inner lumen of the vessel, to a targeted site for implantation in the vessel.

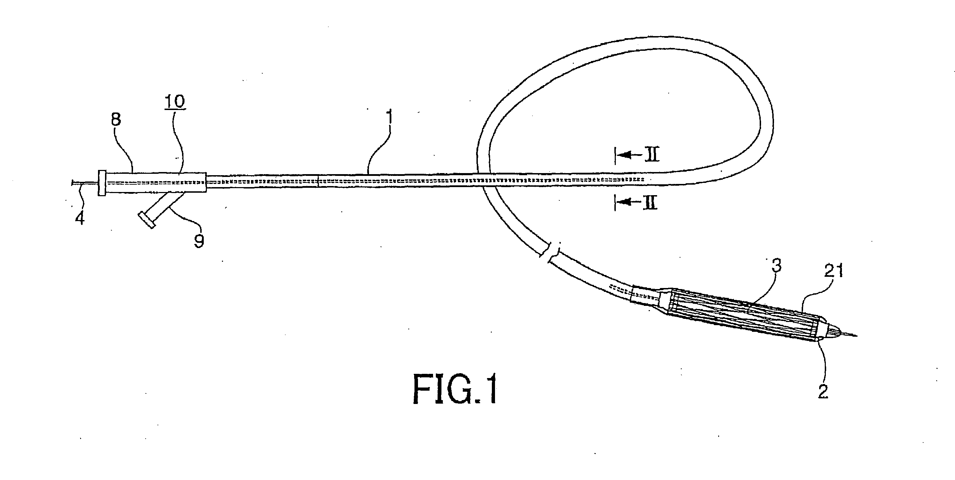

The device for delivery of a stent for the vessel, according to the present invention, includes a catheter 1, introduced into the vessel of a living body, and a balloon 2, adapted for being expanded by a fluid, supplied to the catheter 1, on the outer periphery of the distal end of the catheter, as shown in Fig.1. On this balloon 2 is retained a stent for a vessel 3, which is implanted in the vessel of the living body, such as blood vessel, trachea, bile duct or the urethra, for supporting the lumen of the vessel from an inner side.

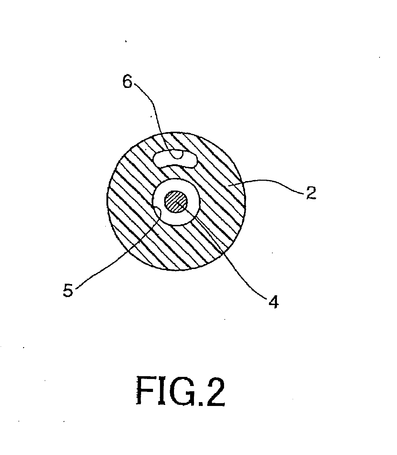

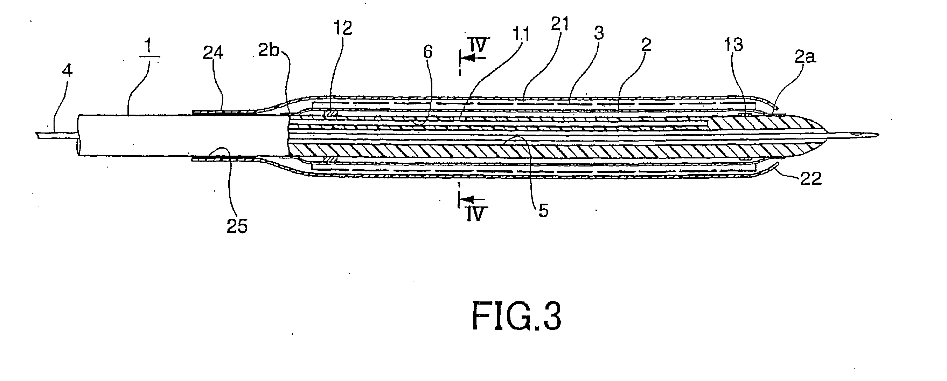

Initially, the catheter 1, holding the stent for the vessel 3, will be explained. The catheter 1 is formed of a flexible polymer material, such as polyethylene, such that the catheter may be introduced into the vessel of the living body as it adapts itself to the shape of the vessel. Referring to Figs.2 and 3, the catheter 1 is provided with a bore 5 in which to insert a guide wire 4 used for guiding the catheter being introduced into the vessel, and a passageway 6 for a fluid, such as a contrast medium, used for expanding the balloon 2 attached to the distal end of the catheter 1. It is noted that the bore 5 in which to insert the guide wire is formed for passing through from the proximal end to the distal end of the catheter 1, whilst the passageway 6 for the fluid is closed just short of the distal end of the catheter 1 as in Fig. 3.

To the proximal end of the catheter 1 is mounted a Y-shaped connector 10, as shown in Fig.1. This Y-shaped connector includes a guide wire guide portion 8, for guiding the guide wire 4 being inserted into the bore 5, and a fluid supply fixture connecting portion 9, to which is connected a fluid supply fixture for supplying a fluid to the balloon 2 via passageway 6.

To the distal end of the catheter 1 is mounted the balloon 2 used for expanding the stent for the vessel 3, mounted to the catheter 1, as shown in Fig.3. The balloon 2 is formed to a tube from, for example, polyethylene (PE), polyolefinic copolymers (POCs) or polyethylene terephthalate (PET). This balloon 2 is mounted to cover up the outer peripheral surface of the distal end of the catheter 1, and has both ends 2a, 2b bonded to the outer peripheral surface of the catheter 1 with, for example, an adhesive, whereby the balloon is fixedly mounted as one to the catheter 1. In an initial state, in which the balloon 2 has been mounted to the catheter 1, the balloon is folded along the outer peripheral surface of the catheter 1.

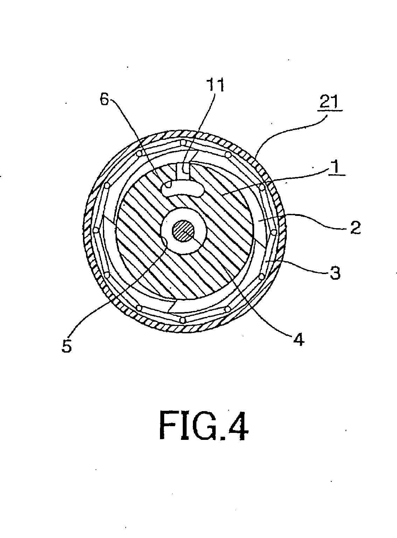

In the portion of the catheter 1, where the balloon 2 is mounted to the catheter, there is bored a communication opening 11 for communication with the passageway 6 for the fluid, as shown in Figs.3 and 4. The contrast medium, supplied via passageway 6 for the fluid, is charged into the inside of the balloon 2, via communication opening 11, for dilating the balloon 2. In the portion of the catheter 1, where the balloon 2 is mounted to the catheter, there are mounted radiopaque potions 12, 13, formed of a material impermeable to X-rays. These radiopaque portions 12, 13 are formed by mounting fine wires of metal, as material impermeable to X-rays, to the outer periphery of the catheter 1. The radiopaque portions 12, 13, provided to the catheter 1, are mounted in the vicinity of both ends 2a, 2b of the balloon 2. Thus, the positions of insertion into the blood vessel of the stent for the vessel 3, mounted on the balloon 2, may be confirmed from outside the living body, with the aid of the radiopaque portions 12, 13.

On the balloon 2, mounted to the catheter 1, there is mounted the stent for the vessel 3, implanted in the vessel, for example, the blood vessel, of the living body.

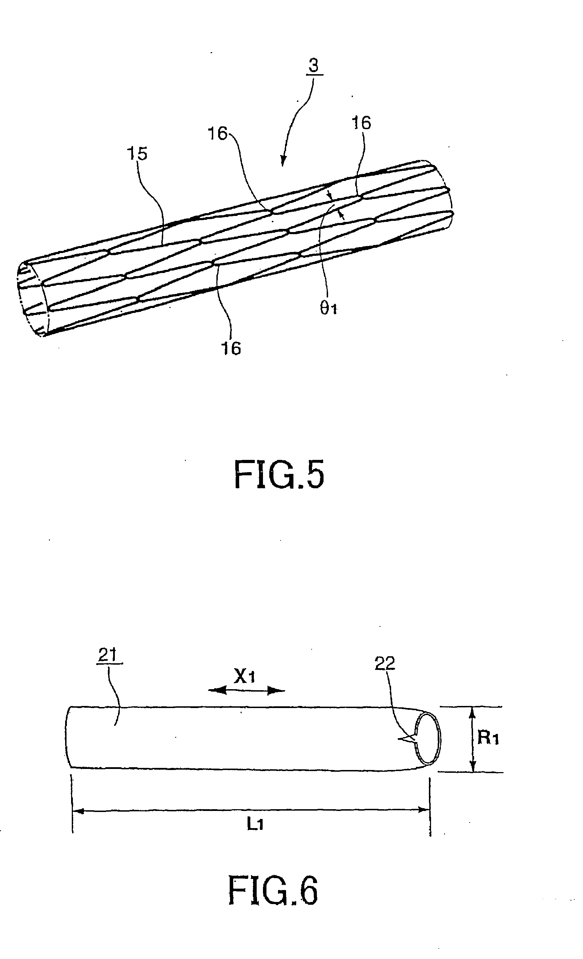

The stent for the vessel 3, used in the present invention, is formed of a biodegradable polymer to a tube form, and exhibits a self-expandable function. An exemplary configuration of the stent for the vessel 3 is shown in Fig.5.

The stent for the vessel 3, shown in Fig.5, is formed to a tube, using a yarn 15 formed of a biodegradable polymer. That is, the stent for the vessel 3 is formed to a tube form, in particular, to a cylindrical configuration, by spirally winding the yarn 15 of the biodegradable polymer, as the yarn is bent in a zigzag design, so that the yarn will present concatenated vee shapes, as shown in Fig.5.

The so formed stent for the vessel 3 may be contracted or expanded in diameter by displacing an angle of bend θ₁, with a point of bend 16 of the yarn 15 as a displacing point.

Meanwhile, the biodegradable polymer of the yarn 15 that may be used may be enumerated by aliphatic polyesters, aliphatic acid anhydrides, aliphatic polycarbonates, polyphosphasen, or a copolymer containing at least one of these substances.

In more detail, one or more of the materials, selected from the group of poly-L lactic acid (PLLA), polyglycolic acid, polyglactin, polydioxanone, polyglyconate, ε-caprolactone, a polylactic acid-ε-caprolactone copolymer and a polyglycolic acid-ε-caprolactone copolymer, may be used as the biodegradable polymer.

The stent for the vessel 3, formed from the yarn 15 of the biodegradable polymer, has the self-expanding properties, and hence is of such properties that, even if it is contracted in diameter by bending so as to reduce the angle of bend θ₁ of the points of bend 16, the angle of bend θ₁ is increased when the stent for the vessel is warmed by body temperature, with the points of bend 16 being then opened to a wider angle to expand the diameter of the stent for the vessel 3.

The stent for the vessel 3, formed to a tube, is mounted on the balloon 2, mounted on the catheter 1 in a diameter-contracted state, as shown in Fig.3. At this time, the balloon 2 is not expanded and is in a folded position, as shown in Fig.4. The portion of the catheter 1, carrying the balloon 2, is formed to an outer diameter approximately equal to the inner diameter of the stent for the vessel 3 in the diameter-contracted state, in order that the stent for the vessel 3 contracted in diameter will be mounted in close contact with the balloon 2.

Since the stent for the vessel 3 is mounted in close contact with the balloon 2, mounted on the catheter 1, the stent for the vessel may be expanded quickly in keeping with inflation of the balloon 2.

The stent for the vessel 3, mounted on the balloon 2, as described above, is covered up with a stent holding member 21, formed of a polymer, as shown in Fig.3.

This stent holding member 21 is used for holding the stent for the vessel 3, mounted in the diameter-contracted state on the folded balloon 2, in this diameter-contracted state. Hence, the stent holding member 21 is formed to a tube form of an internal diameter R₁ sufficient to keep the stent for the vessel 3, mounted in the diameter-contracted state on the deflated balloon 2, in this diameter-contracted state, as shown in Fig.6.

Since the stent holding member 21 holds the stent for the vessel 3, having the force of self-expansion, in the diameter-contracted state, the stent holding member is preferably formed of a polymer material which may not be readily subjected to expansion or contraction. Moreover, when the stent for the vessel 3 is delivered within the vessel, the stent holding member 21 is directly contacted with the inner wall of the vessel, so that, for assuring smooth delivery, the stent holding member is desirably formed of a highly lubricious polymer material. Thus, according to the present invention, the stent holding member 21, formed of PTFE (polytetrafluoroethyelene), as a highly lubricious polymer material, is used. That is, PTFE molded to a tube or a film-shaped PTFE formed to a tube, is used as the stent holding member 21.

Of course, the material that makes up the stent holding member 21 is not limited to PTFE.

The stent holding member 21, formed to a tube from a polymer material, such as PTFE, is drawn along the longitudinal direction perpendicular to an internal diameter R₁ thereof, as indicated by an arrow X₁ shown in Fig.6. That is, with the stent holding member 21, drawn in the longitudinal direction, the polymer molecules, which make up this stent holding member 21, are oriented in the long-axis direction.

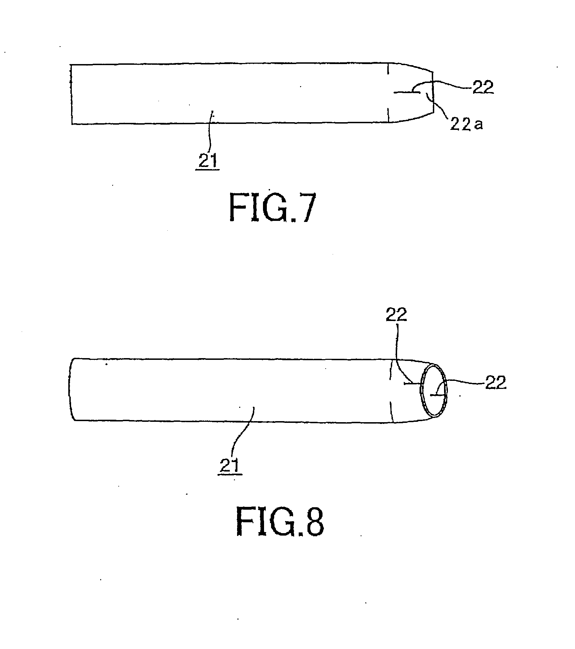

One end of the stent holding member 21 is formed with a tearing assisting portion 22, as shown in Fig.6. The tearing assisting portion 22 guides an initially torn location of the stent holding member 21 when the force of dilation is applied to the stent holding member 21 from its inner side. Thus, the tearing assisting portion is formed by providing one end of the stent holding member 21 with a vee-shaped slit shown in Fig.6 or with a linear incision shown in Fig.7.

The tearing assisting portion 22 may be provided in two or more locations, as shown in Fig.8, instead of in one location. In case plural tearing assisting portions 22 are provided, they are preferably provided at equiangular positions in the circumferential direction of the tubular stent holding member 21. By providing these plural tearing assisting portions 22, the stent holding member 21 may be torn positively. That is, by providing these plural tearing assisting portions 22, one of them may reliably initiate the tearing, at the time of tearing the stent holding member 21, such that the stent holding member may reliably be torn beginning from the tearing-initiating one of the tearing assisting portions 22.

Since the stent holding member 21 is drawn in the longitudinal direction, it is readily torn in the same direction if once the tearing commences with the tearing assisting portion 22 as a guide. Thus, the tearing assisting portion 22 needs only to guide the tearing so that, when the stent holding member 21 is initially expanded in diameter, the tearing will commence from the tearing assisting portion, such that it is sufficient only to provided an only small slit in a portion of the stent holding member 21.

Meanwhile, when the tearing assisting portion 22 is formed by forming a linear slit, shown in Fig.7, the tearing assisting portion 22 may be formed as the one end of the stent holding member 21 is opened, that is, as the slit is formed beginning from the one end of the stent holding member 21. However, the one end side of the stent holding member 21 may be closed by a readily tearable connecting portion 22a, as shown in Fig.7. By keeping intact the one end of the stent holding member, carrying the tearing assisting portion 22, by the connecting portion 22a, the stent holding member 21 may be prevented from being torn inadvertently. That is, the stent holding member 21 may be prevented from being torn inadvertently, beginning from the tearing assisting portion 22, such as during shelving, such that the stent for the vessel 3 may reliably be maintained in the diameter-contracted state.

The stent holding member 21 is used for preventing the stent for the vessel 3, mounted in the diameter-contracted state on the balloon 2, from becoming self-expanded in the course of introduction into the vessel to perform an inadvertent movement on the balloon 2 which is in the contracted state. In the present embodiment, the stent holding member 21 is formed to a length L₁ sufficient to cover the entire length of the stent for the vessel 3 mounted on the balloon 2.

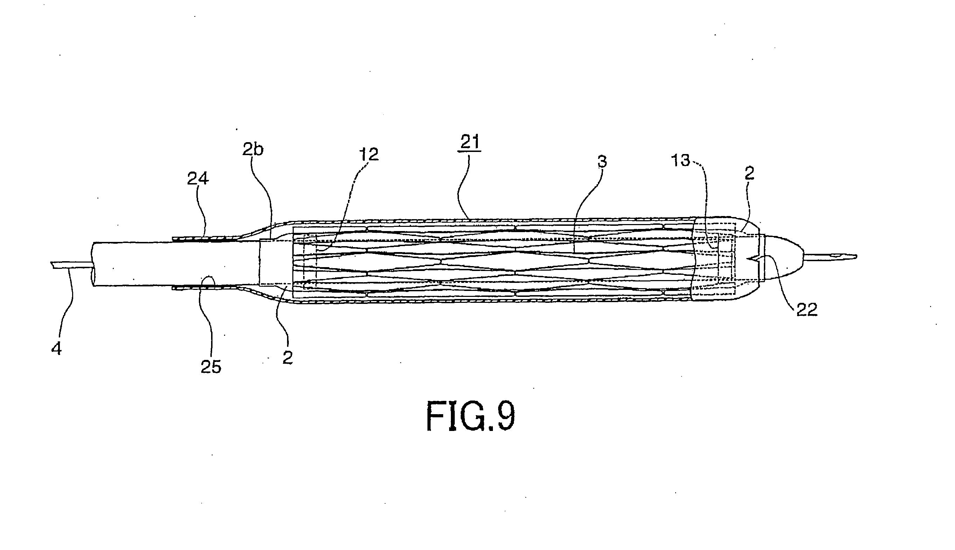

The distal end of the stent holding member 21, provided with the tearing assisting portion 22, is contracted in diameter, as shown in Fig.9. That is, the distal end of the stent holding member 21 is contracted in diameter, along the shape of the balloon 2 mounted in the folded contracted state on the catheter 1, thus assuring facilitated insertion of the stent for the vessel 3 into the vessel of the living body.

For mounting the stent for the vessel 3 on the balloon 2 in the diameter-contracted state, as the stent for the vessel 3 is held in the diameter-contracted state, using the stent holding member 21, constructed as described above, the stent for the vessel 3 in the diameter-contracted state is introduced into the inside of the stent holding member 21.

The stent holding member 21 is then mounted on the balloon 2, with the distal end thereof, provided with the tearing assisting portion 22, lying towards the distal end of the catheter 1, as shown in Fig.9. At this time, the proximal end 24 of the stent holding member 21, opposite to its end carrying the tearing assisting portion 22, is located on the catheter 1, and is secured to the outer peripheral surface thereof. That is, the proximal end 24 proves a fixing part of the stent holding member to the catheter 1. This fixing of the stent holding member 21 to the catheter 1 is done by bonding with an adhesive 25.

The fixing of the stent holding member 21 to the catheter 1 may also be done by winding a yarn around the outer surface of the proximal end 24.

In this manner, the stent holding member 21 may be fixed to the catheter 1 and thereby prevented from becoming detached from the catheter 1. In addition, the stent for the vessel 3 may reliably be in the diameter-contracted state. Even when the stent holding member 21 is torn in the longitudinal direction, along the tearing assisting portion 22, the stent holding member may be maintained as one with the catheter 1, without becoming released therefrom, because the distal end of the stent holding member is fixed to the catheter. Thus, the stent holding member may reliably be taken out from within the vessel, along with the catheter 1, after implanting the stent for the vessel 3 in the vessel.

Meanwhile, in implanting the stent for the vessel in the vessel of the living body, it is necessary to prevent air from being intruded into the inside of the vessel, no matter what sort of the device for delivery of a stent for the vessel is used.

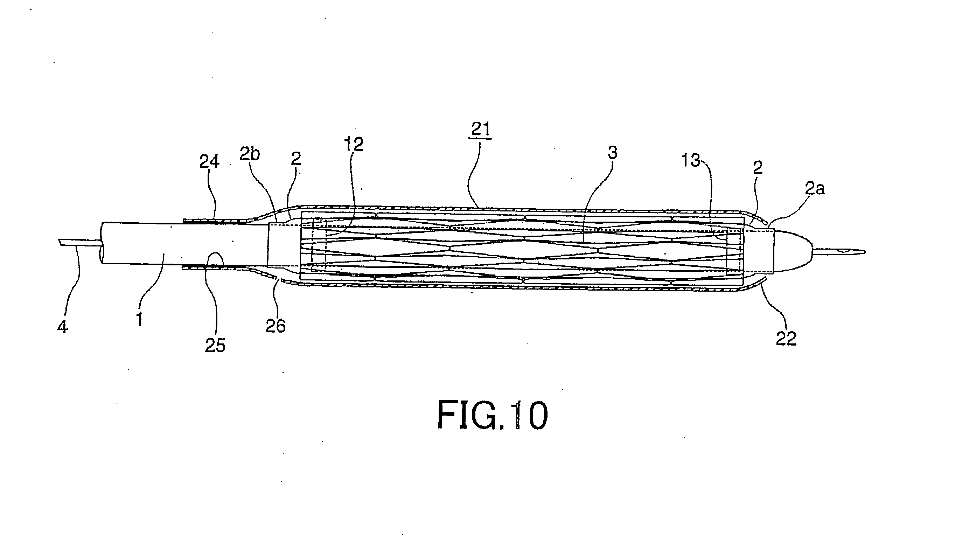

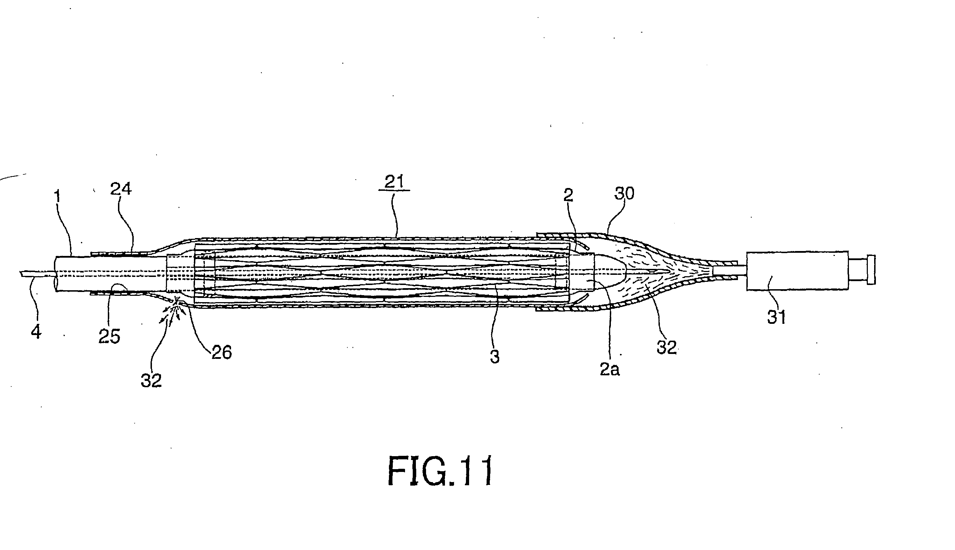

With the device for delivery of a stent for the vessel, according to the present invention, it is necessary to positively prevent air from being left within the stent holding member 21 which supports the stent for the vessel 3 in an overlying fashion. Thus, with the device for delivery of a stent for the vessel, according to the present invention, it is necessary to carry out the processing for removing air left in the inside of the stent holding member 21 just before the stent implantation. In order to carry out this air removing operation with ease, an air-vent through-hole 26 is bored in the vicinity of the proximal end 24 of the stent holding member 21 secured to the catheter 1 as shown in Fig. 10. By providing the stent holding member 21 with the through-hole 26 in this manner, it is possible to inject a liquid, such as physiological saline, from the distal end of the stent holding member 21, mounted to the catheter 1, to permit the liquid to exit from the through-hole 26, by way of performing air venting from within the stent holding member 21. In removing air in this manner, a syringe 31 is mounted via a flash adapter 30 to the distal end of the stent holding member 21, mounted on the catheter 1, to introduce a liquid 32, such as physiological saline, via this syringe 31, as shown in Fig.11. The liquid 32, thus introduced into the inside of the stent holding member 21, is discharged to outside the stent holding member 21, via through-hole 26, to remove air from within the stent holding member 21.

The state in which, with the device for delivery of a stent for the vessel, constructed as described above, the stent for the vessel 3 is implanted within the vessel, will now be explained.

For implanting the stent for the vessel 3 in a targeted implant position in the vessel, the catheter 1, mounted with the stent for the vessel 3, is introduced into the vessel, with its distal end, mounted with the stent for the vessel 3, as a leading end. Since the stent holding member 21, covering up the stent for the vessel 3, is formed of PTFE, which is a highly lubricious material, the catheter may smoothly be introduced without producing any marked friction between it and the vessel wall.

Moreover, since the stent for the vessel 3 is covered substantially over its entire length by the stent holding member 21, the stent for the vessel 3 is kept in its diameter-contracted state, without self-expansion, even when the stent for the vessel 3 is inserted into the vessel and warmed by body temperature of the living body. Since the stent holding member 21 has its proximal end 24 secured to the catheter 1, the stent holding member is delivered reliably as one with the catheter 1, as the catheter is introduced into the vessel, so that the stent for the vessel 3 may be retained on the balloon 2 without performing inadvertent movements on the balloon mounted on the catheter 1.

The catheter 1 is introduced into the vessel until the stent for the vessel 3 has been delivered to the targeted implant site in the vessel.

Meanwhile, the location of insertion of the stent for the vessel 3 may be confirmed by the radiopaque portions 12, 13 provided on both ends of the balloon 2.

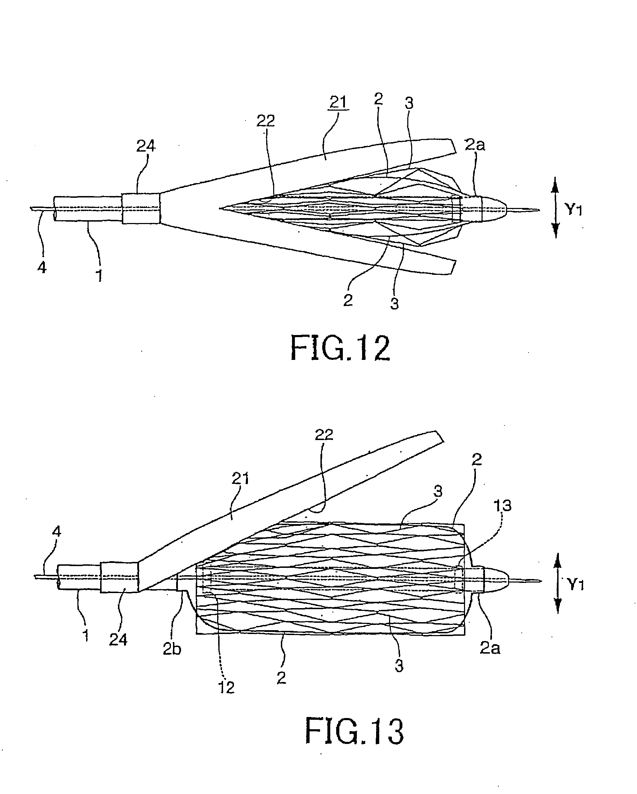

When the stent for the vessel 3 has been delivered to a targeted implant site in the vessel, the catheter 1 is fixed, and a liquid, such as contrast medium, is supplied to the passageway 6 for the fluid via a fluid supply fixture connected to the fluid supply fixture connecting portion 9 of the Y-shaped connector 10. The liquid, supplied to the passageway 6 for the fluid, is supplied via communication opening 11 into the inside of the balloon 2 to dilate the balloon. When the balloon 2 is expanded in the direction indicated by arrow Y₁ shown in Fig.12, the stent for the vessel 3 is expanded in diameter, in keeping with inflation of the balloon 2, such that the force of expansion is applied to the stent holding member 21 which covers up the stent for the vessel 3. At this time, the force of tearing acts the tearing assisting portion 22. When the balloon 2 is expanded further from the state in which the tearing force has acted on the tearing assisting portion 22, the stent holding member 21 is torn from the distal end towards the proximal end 24, along the tearing assisting portion 22, as shown in Fig.12. Since the stent holding member 21 has been drawn from the distal end towards the proximal end 24, along the longitudinal direction, it is torn along the longitudinal direction from the distal end towards the proximal end 24.

In case there are provided a plural number of the tearing assisting portions 22 in the stent holding member 21, the stent holding member is torn with one or more tearing assisting portion(s) 22 as the guide for tearing.

When the balloon 2 is expanded in the direction indicated by arrow Y₁ in Fig.12, such as to tear the stent holding member 21, the stent for the vessel 3 is also expanded to keep pace with inflation of the balloon 2. When the balloon 2 is expanded to its maximum extent, as shown in Fig. 13, the stent for the vessel 3 is expanded to a state in which the stent for the vessel supports the inner wall of the vessel.

Meanwhile, even if the balloon 2 has been expanded until the stent for the vessel 3 is expanded to a size capable of supporting the inner wall of the vessel, the stent holding member 21 is not torn throughout its entire length, and remains affixed to the catheter 1, because the proximal end 24 of the stent holding member is secured to the catheter 1, as shown in Fig. 13.

After the stent for the vessel 3 is expanded in diameter such that it is capable of supporting the inner wall of the vessel, the liquid supplied through the fluid supply fixture is sucked to deflate the balloon 2. At this time, the stent for the vessel 3 is maintained in its expanded state to support the inner wall of the vessel. If then the catheter 1 is extracted from within the vessel, the balloon 2 and stent holding member 21 are released from the stent for the vessel 3 and drawn outward from the living body to complete the implantation of the stent for the vessel 3 within the vessel.

Since the stent holding member 21 is formed of a highly lubricious material, such as PTFE, it can be smoothly extracted from a space between the expanded stent for the vessel 3 and the inner wall of the vessel to prevent the stent for the vessel 3 from migrating from the implant position.

Also, when the stent for the vessel 3 is expanded in diameter, the stent holding member 21 is torn along the tearing assisting portion 22, such that at least a portion of the stent for the vessel 3 is exposed to outside the stent holding member 21 to directly support the inner wall of the vessel. Thus, when the catheter 1 is extracted from the vessel, the stent for the vessel 3 does not migrate in keeping with the stent holding member 21, but remains implanted in a target implant position.

In the above-described device for delivery of a stent for the vessel, the stent holding member 21 is affixed to the catheter 1. Alternatively, with the device for delivery of a stent for the vessel, according to the present invention, the stent holding member 21 may be mounted for movement relative to the catheter 1.

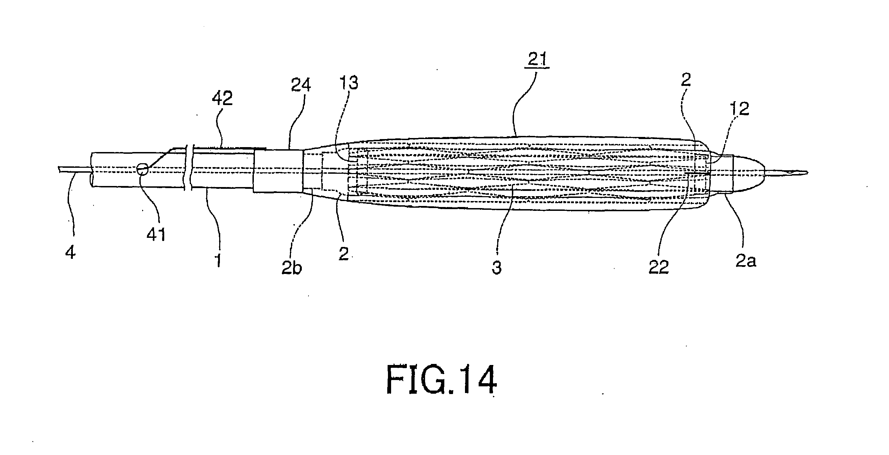

In Fig.14, there is shown an embodiment of the present invention in which the stent holding member 21 is mounted for movement relative to the catheter 1. The present device for delivery of a stent holds the stent for the vessel 3, mounted on the balloon 2, without securing the proximal end of the stent holding member 21 to the catheter 1, as shown in Fig.14. This stent holding member 21 is dimensioned such that, when the stent holding member, which has enclosed the stent for the vessel 3, is mounted on the balloon 2, the stent holding member is mounted in tight contact with the balloon 2 in the folded contracted state, so that it may not be easily detached from the balloon 2, as shown in Fig.14.

This stent holding member 21 is mounted to the balloon 2 which is in the folded and contracted state, as the stent holding member has enclosed the stent for the vessel 3, which is in the diameter-contracted state. The stent holding member is then mounted on the balloon 2. This holds the stent for the vessel 3 in the state mounted on the balloon 2.

The diameter-contracted proximal end 24 of the stent holding member 21, mounted to the balloon 2 as the stent holding member has enclosed the stent for the vessel 3, is connected to a maneuvering wire 42, inserted through the catheter 1 and extracted to outside through an extraction opening 41, bored in a mid portion of the catheter 1, as shown in Fig. 14.

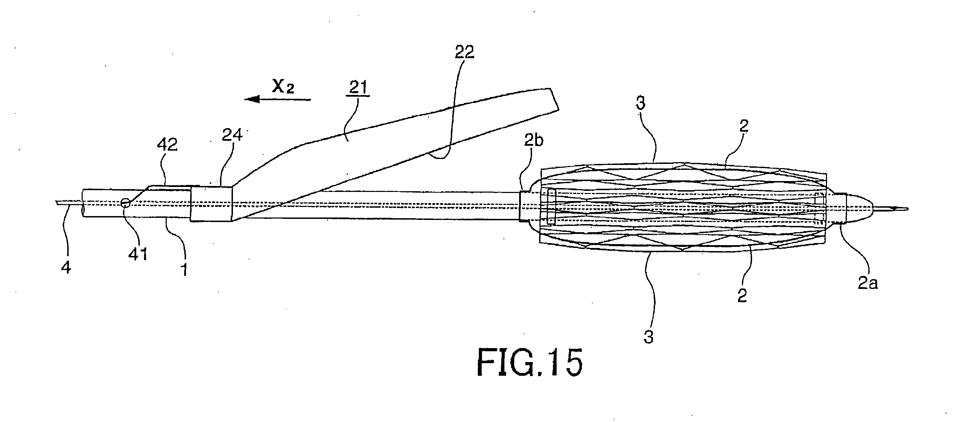

If, in the device for delivery of a stent for the vessel, constructed as described above, the stent for the vessel 3 is delivered to a targeted implant position in the vessel of the living body, and the balloon 2 is expanded, the stent holding member 21 is torn along the tearing assisting portion 22, in keeping with inflation of the balloon 2. If, when the stent for the vessel 3 is expanded sufficiently in diameter, the maneuvering wire 42 is acted on in a direction indicated by arrow X₂ in Fig.15, the stent holding member 21 becomes disengaged from the stent for the vessel 3, as a result of which the stent for the vessel 3 directly supports the inner wall of the vessel.

Since the stent holding member 21 may be disengaged from the stent for the vessel 3, as the balloon 2 is inflated and the stent for the vessel 3 is expanded in diameter, the stent for the vessel 3 may reliably be implanted in the targeted implant position. Stated differently, the stent holding member 21 may be released as the stent for the vessel 3 is supported by the inflated balloon 2. Moreover, if it becomes necessary to expand the balloon 2 again to expand the diameter of the stent for the vessel 3, the stent for the vessel 3 may be expanded in diameter in the absence of the stent holding member 21.

It should be noted that the stent holding member 21 may be disengaged from the stent for the vessel 3, expanded in diameter, after deflating the balloon 2. In the device for delivery of a stent for the vessel, according to the present invention, the stent holding member 21 has already been torn along the tearing assisting portion 22 when the stent for the vessel 3 is expanded in diameter, hence, at least a portion of the stent for the vessel 3 directly supports the inner wall of the vessel at the torn portion of the stent holding member 21. Thus, even if the balloon 2 is deflated, the stent for the vessel 3 supports the inner wall of the vessel, and hence the implant position thereof does not migrate. Consequently, the stent holding member 21 may be disengaged from the stent for the vessel 3 after deflating the balloon 2.

With the device for delivery of a stent, constructed as described above, the stent for the vessel 3, mounted on the balloon 2 of the catheter 1, and having the force for self-expansion, may reliably be delivered to and implanted at the targeted implant position in the vessel.

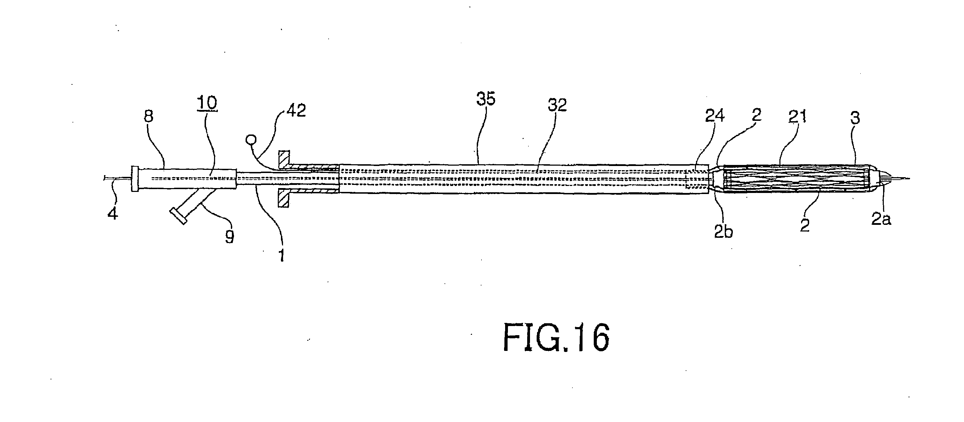

Meanwhile, with the device for delivery of a stent for the vessel, in which the stent holding member 21 may be pulled out using the maneuvering wire 42, the catheter 1 may be inserted into a protective sheath 35, as shown in Fig.16. In the device for delivery of a stent for the vessel, shown in Fig.16, the maneuvering wire 42 is connected to the stent holding member 21 which has covered up the stent for the vessel 3, and is passed through and led outward from the protective sheath 35. With the use of the protective sheath 35, the maneuvering wire 42 need not be passed through the catheter 1, thus simplifying the structure of the catheter 1. Moreover, since the maneuvering wire 42 may be prohibited from being exposed to outside and having direct contact with the inner wall of the vessel, the stent for the vessel 3 may be delivered in safety within the vessel, along with the stent holding member 21.

Furthermore, by introducing the proximal end 24 of the stent holding member 21, disposed on the catheter 1, into the distal end of the protective sheath 35, as shown in Fig.16, the stent holding member 21 may be reliably pulled into the inside of the protective sheath 35 by the operation of pulling outward of the maneuvering wire 42.

With the device for delivery of a stent, shown in Fig.16, since the stent holding member 21, torn as a result of inflation of the balloon 2, is introduced into the protective sheath 35 and pulled outward in this state from the living body, it is possible to prevent thrombus from being formed with the stent holding member 21 as nucleus.

The device for delivery of a stent for the vessel according to the present invention, described above, is simplified in structure and, with the use of the present device, the stent for the vessel, which is formed of a biodegradable material, and has the self-expanding properties, and which nevertheless is in need of expansion with the balloon, may be prohibited from descent from the catheter and may be correctly implanted in the targeted site in the vessel. In addition, the stent for the vessel may be delivered in safety in such a state that the stent for the vessel does not damage the vessel, such as blood vessel.

The present invention is not limited to the above embodiments explained with reference to the drawings and, as may be apparent to those skilled in the art, various changes or substitutions by equivalents may be attempted without departing from the scope of the invention.

### Industrial Applicability

With the device for delivery of a stent for the vessel, according to the present invention, described above, the stent for the vessel, formed of a biodegradable polymer and hence afforded with the self-expanding properties, and which nevertheless is in need of expansion with the balloon, may be reliably implanted at a targeted site in the vessel. Moreover, the stent for the vessel may be inserted in safety into the vessel, such as a blood vessel, as damage to the vessel is suppressed to a minimum.

## Claims

1. A device for delivery of a stent for the vessel comprising:
a catheter for insertion into the vessel of a living body;
a balloon mounted on an outer peripheral surface of the distal end side of said catheter and inflatable with a fluid supplied to said catheter;
a stent for the vessel mounted on said balloon in a diameter-contracted state, said stent being formed of a biodegradable polymer and having self-expanding properties; and
a stent holding member formed of a polymer material to a tube form for holding said stent for the vessel on said balloon, and configured for covering at least a portion of said stent for the vessel from said catheter;
said stent holding member having been drawn in the longitudinal direction and being provided with a tearing assisting portion at a distal end thereof located towards the distal end of said catheter.

2. The device for delivery of a stent for the vessel according to claim 1, wherein said tearing assisting portion is a slit provided to the distal end side of said stent holding member.

3. The device for delivery of a stent for the vessel according to claim 1, wherein the distal end of said tearing assisting portion is closed by a connecting portion.

4. The device for delivery of a stent for the vessel according to claim 1, wherein said tearing assisting portion is a slit formed for extending along the drawing direction of said stent holding member.

5. The device for delivery of a stent for the vessel according to claim 1, wherein said stent holding member is formed of PTFE (polytetrafluoroethylene).

6. The device for delivery of a stent for the vessel according to claim 1, wherein the proximal side of said stent holding member, located on said catheter, is secured to said catheter.

7. The device for delivery of a stent for the vessel according to claim 6, wherein an air-vent through-hole is bored in the proximal side of said stent holding member secured to said catheter.

8. The device for delivery of a stent for the vessel according to claim 1, wherein said stent holding member covers up the entire length of said stent for the vessel.

9. The device for delivery of a stent for the vessel according to claim 8, wherein the distal end of said stent holding member, provided with said tearing assisting portion, is contracted in diameter so as to be tightly contacted with said balloon.

10. The device for delivery of a stent for the vessel according to claim 1, wherein said stent holding member is connected to a yarn passed through said catheter so as to be pulled out partway from said catheter, and wherein said stent holding member may be released from the stent for the vessel by pulling said yarn outward from said catheter.

11. The device for delivery of a stent for the vessel according to claim 1, wherein said stent for the vessel is formed of a yarn of a biodegradable polymer to a tube form.
